# FASCICULE DE BREVET EUROPEEN

(11) **EP 3 417 258 B1**
(45) Date de publication et mention de la délivrance du brevet: **19.11.2025**
(21) Numéro de dépôt: 17707777.3
(22) Date de dépôt: 15.02.2017
(51) Int. Cl.: G01L 5/00, A61B 5/22

(54) **DISPOSITIF DE MESURE D'UNE FORCE**
KRAFTMESSVORRICHTUNG
FORCE MEASURING DEVICE

(30) Priorité: 19.02.2016 FR 1651395
(43) Date de publication de la demande: 26.12.2018
(73) Titulaire: Association Institut de Myologie, 75013 Paris (FR)
(72) Inventeur: HOGREL, Jean-Yves, 92120 Montrouge (FR)
(74) Mandataire: IPAZ
(86) Numéro de dépôt international: PCT/EP2017/053428
(87) Numéro de publication internationale: WO 2017/140738

(56) Documents cités:
- EP-A2- 0 083 568
- EP-A2- 0 083 568
- FR-A1- 2 988 838
- FR-A1- 2 988 838
- JP-A- S58 190 727
- US-A- 3 577 779
- US-A- 3 680 386
- US-A- 3 680 386
- US-A- 5 090 421
- MECMESIN: "Myometer - Operator Manual", 1 November 2014 (2014-11-01), XP055307716, Retrieved from the Internet <URL:http://www.mecmesin.com/documents/manuals/431-378-03 Myometer.pdf> [retrieved on 20161005]

## Description

### Domaine technique

La présente invention concerne un dispositif de mesure d'une force.

L'invention fait partie du domaine de la métrologie. Le domaine de l'invention est plus particulièrement, mais de manière non limitative, celui de la mesure de force sur un patient potentiellement très faible, par exemple de force d'extension ou flexion d'un genou ou de l'abduction d'une épaule ou de la flexion d'un coude.

### Etat de la technique antérieure

On connaît des dispositifs de mesure de force sur des patients potentiellement très faibles.

Parmi ces dispositifs, on connait les dispositifs de dynamométrie fixe comprenant un capteur de force disposé entre deux sangles dont l'une est fixée à un point fixe immobile (le plus souvent un point d'une structure murale) et l'autre est reliée au patient moyennant un strap ergonomique adapté à la taille du patient et à la taille du membre testé.

Un problème technique posé par ce genre de dispositif est que certains patients sont tellement faibles qu'ils ne peuvent pas mettre le capteur et la sangle en tension.

Le but de la présente invention est de résoudre ce problème.

Le document FR 2 988 838 décrit un système de mesure d'une force de préhension palmaire.

Le document EP 0 083 568 A2 décrit un testeur de muscle manuel portable.

Le document US 3 680 386 décrit un dispositif de diagnostic de thérapie physique.

Le document JP S58 190727 décrit une balance de cuisine. Le document US3577779 décrit un capteur de force comportant des moyens de fixation par un trou fileté dans la base.

### Exposé de l'invention

Cet objectif est atteint avec un dispositif de mesure d'une force selon la revendication 1.

Le capteur a de préférence une précision inférieure ou égale à 0,5N.

Le capteur a de préférence une plage de mesure d'au moins 0N à 900 N.

Le membre de mesure peut être muni d'un pas de vis agencé pour fixer au membre de mesure un élément extérieur s'étendant le long de l'axe de mesure à travers le trou de passage.

Le dispositif selon l'invention peut comprendre au moins une carte électronique agencée pour :
- une mise en forme d'un signal électronique en provenance du capteur en données de mesure de force et/ou
- une alimentation électrique du capteur et/ou d'autres éléments du dispositif, et/ou
- une communication, vers l'extérieur du dispositif, de données de mesure de force en provenance du capteur, et/ou
- un stockage de données de mesure de force en provenance du capteur.

L'au moins une carte électronique s'étend de préférence :
- parallèlement à l'axe de mesure, et/ou
- sous le capteur et sous les moyens de fixation.

Suivant encore un autre aspect de l'invention, il est proposé un ensemble comprenant :
- un dispositif selon l'invention, dans lequel l'au moins une carte électronique est agencée pour une communication sans fil, vers l'extérieur du dispositif, de données de mesure de force en provenance du capteur, et
- un moyen déporté agencé pour afficher les données de mesure et/ou un traitement des données de mesure, ledit moyen déporté étant relié audit dispositif par une liaison non filaire.

### Description des figures et modes de réalisation

D'autres avantages et particularités de l'invention apparaîtront à la lecture de la description détaillée de mises en œuvre et de modes de réalisation nullement limitatifs, et des dessins annexés suivants :
- la figure 1 est une vue de dessus d'un premier mode de réalisation de dispositif selon l'invention, qui est le mode de réalisation préféré de l'invention,
- la figure 2 est une vue en perspective d'une face latérale du premier mode de réalisation de dispositif selon l'invention,
- la figure 3 est une vue en perspective d'une autre face latérale du premier mode de réalisation de dispositif selon l'invention, et
- la figure 4 est une vue en perspective du dessous du premier mode de réalisation de dispositif selon l'invention, sans sa plaque de fond pour rendre visible l'intérieur du dispositif selon l'invention.

Ces modes de réalisation étant nullement limitatifs, on pourra notamment considérer des variantes de l'invention ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

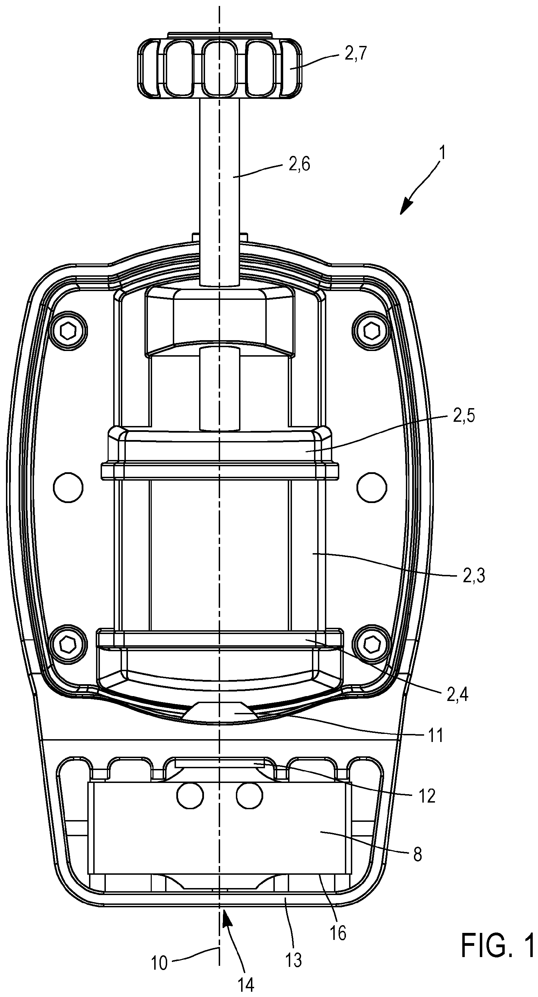

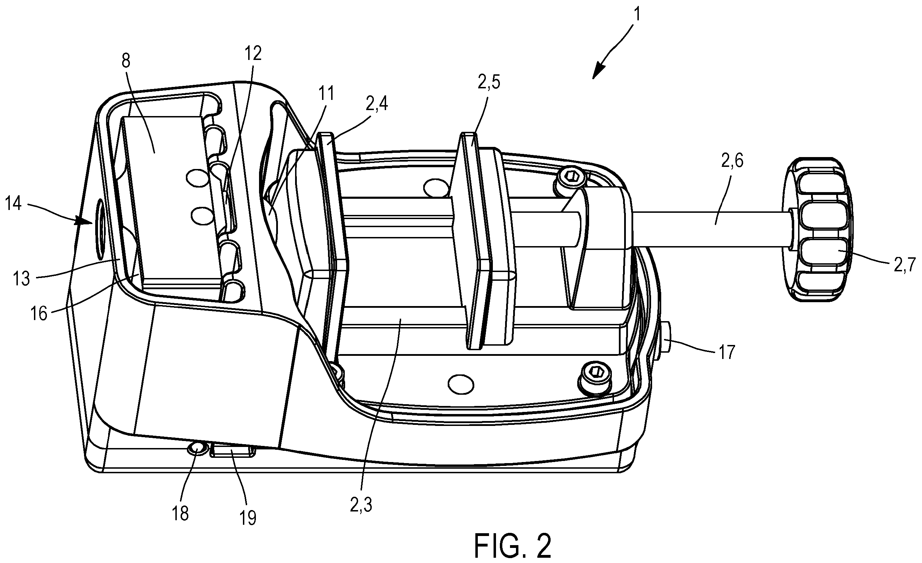

On va tout d'abord décrire, en référence aux figures 1 à 4, un premier mode de réalisation de dispositif 1 selon l'invention.

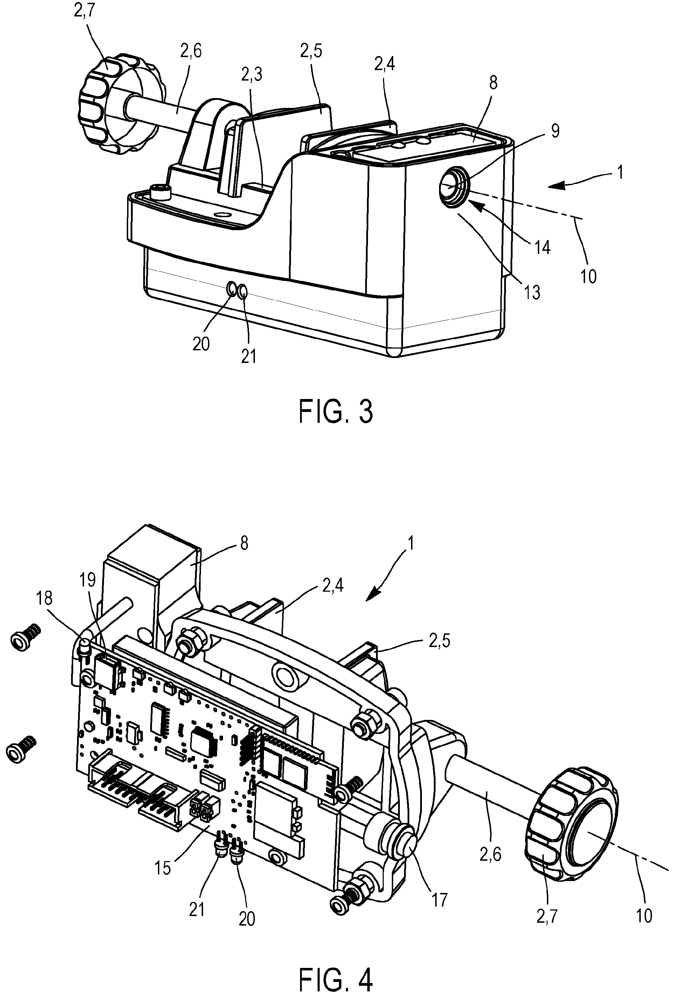

Le dispositif 1 de mesure d'une force comprend :
- des moyens de fixation 2, agencés pour fixer le dispositif 1 à un support, par exemple à un lit, à une table quelconque ou une table de clinique, à un plan de Bobath, à une chaise, typiquement à un pied ou cadre ou rebord d'un de ces éléments,
- un capteur 8, comprenant un membre de mesure 9 (visible sur la figure 3) agencé pour se déplacer (par rapport au bâti du dispositif 1) selon un axe de mesure 10 et uniquement selon cet axe de mesure 10, ledit capteur 8 étant agencé pour mesurer une force exercée sur le membre de mesure 9 en fonction du déplacement de ce membre de mesure 9.

Les moyens de fixation 2 sont agencés pour, lors d'un déplacement du membre de mesure 9 le long de l'axe de mesure 10, exercer sur un support sur lequel ils sont fixés une force moyenne portée par l'axe de mesure 10. Autrement dit, l'axe 10 du capteur 8 est confondu avec l'axe 10 des moyens de fixation 2.

L'invention a pour avantage que le patient n'a pas besoin de tendre une sangle pour compenser le poids du capteur 8, ce qui est particulièrement avantageux pour les patients faibles ayant développé par exemple une pathologie neuromusculaire, neurologique ou cardiovasculaire et/ou pour les personnes âgées

Le membre de mesure 9 du capteur 8 est mobile par rapport au bâti du dispositif 1. Le capteur comprend en outre un boitier immobile par rapport au bâti du dispositif 1, et maintenu solidaire du bâti du dispositif 1 par fixation au bâti entre un écrou 11 et une rondelle 12.

Le capteur 8 comprend une jauge de contrainte ou un dynamomètre.

Le capteur utilisé 8 est un capteur « SML Low Height Load Cell » commercialisé par la société Interface Inc, par exemple de référence SML-200 ou SML-300. Le capteur préféré 8 présente une capacité nominale d'environ 89kgf (200 Ibf).

Le capteur 8 peut être calibré par un procédé tel que décrit dans le brevet FR 2 988 838.

Le capteur 8 a une précision inférieure ou égale à 0,5N et une résolution inférieure ou égale à 0,1N.

Le capteur 8 a une plage de mesure allant jusqu'à au moins 900 N.

Le dispositif 1 est accompagné d'un moyen déporté d'affichage (non illustré sur les figures) agencé pour afficher les données de mesure et/ou un traitement (changement d'échelle, d'unité, réduction du bruit de mesure, etc.) des données de mesure, ledit moyen déporté étant relié audit dispositif 1 par une liaison non filaire (par exemple par Bluetooth ou Wifi).

Ce moyen déporté d'affichage comprend typiquement une tablette (de préférence tactile) ou un PDA (« Personal Digital Assistant » ou « assistant numérique personnel ») ou un smartphone ou un bracelet muni d'un écran (de préférence tactile) et prévu pour être au poignet d'une personne, typiquement au poignet du médecin ou kinésithérapeute ou tout autre personnel médical.

Ce moyen déporté permet de voir en temps réel la valeur de force générée sur le capteur 8 et de mémoriser la valeur maximale de chaque essai.

Ce moyen déporté d'affichage comprend en outre une batterie (autonomie d'une dizaine d'heure lors d'une mesure du capteur 8).

Ce moyen déporté d'affichage comprend en outre des moyens pour mémoriser (typiquement à 50 Hz ou 100Hz) les données de mesure ou le traitement des données de mesure.

Ce moyen déporté d'affichage comprend en outre des moyens (port USB ou autre) pour exporter les données de mesure ou le traitement des données de mesure vers d'autres moyens techniques tel un ordinateur.

Ce moyen déporté d'affichage (par exemple une tablette tactile) est aussi un moyen déporté de commande agencé pour commander certaines fonctions du dispositif 1, notamment le tarage du capteur 8 du dispositif 1.

Les moyens de fixation 2 comprennent des moyens mécaniques de fixation, typiquement des moyens de fixation par serrage. Les moyens de fixation comprennent un étau comprenant :
- deux mâchoires 4, 5 agencées pour serrer le support sur lequel est fixé l'étau, dont une mâchoire fixe 4 par rapport au bâti du dispositif 1 et une mâchoire mobile 5 par rapport au bâti du dispositif 1,
- des rails 3, agencés pour guider un déplacement de la mâchoire mobile 5 par rapport au bâti du dispositif 1,
- une poignée 7 et une tige 6, permettant de déplacer la mâchoire 5 parallèlement à l'axe 10 vers l'avant ou vers l'arrière selon le sens de rotation de la poignée 7 et de la tige 6.

Le dispositif 1 comprend une paroi extérieure 13 (solidaire du bâti du dispositif 1). Le capteur 8 est situé à l'intérieur de la paroi extérieure 13 de sorte que la paroi extérieure 13 est munie d'un trou de passage 14 faisant face au membre de mesure 9 le long de l'axe de mesure 10, le membre de mesure 9 étant situé sur une face 16 du capteur 8 de sorte que cette face 16 ne soit pas en contact de la paroi extérieure 13. Ceci permet d'assurer une meilleure précision de mesure.

Le membre de mesure 9 est muni d'un pas de vis agencé pour fixer au membre de mesure 9 un élément extérieur s'étendant le long de l'axe de mesure 10 à travers le trou de passage 14. Cet élément extérieur est typiquement une tige filetée solidaire d'un crochet ou d'une boucle ou de tout autre système d'accroche relié à une sangle sur laquelle un utilisateur ou patient doit tirer ou à une tige rigide sur laquelle un utilisateur ou patient doit pousser.

Le dispositif 1 comprend au moins une carte électronique 15 agencée pour :
- une mise en forme d'un signal électronique en provenance du capteur 8 en données de mesure de force,
- une alimentation électrique du capteur 8 et des autres éléments du dispositif 1, par une batterie rechargeable ayant une autonomie d'une dizaine d'heures lors d'une mesure par le capteur 8 ; la carte 6 est agencée pour commander une extinction automatique du dispositif 1 et du moyen déporté d'affichage, après 10 minutes de non utilisation du dispositif 1 et de ce moyen déporté,
- une communication sans fil (typiquement par Bluetooth ou via un réseau Wifi), vers l'extérieur du dispositif (plus exactement vers le moyen déporté d'affichage), de données de mesure de force en provenance du capteur,
- un stockage de données de mesure de force en provenance du capteur 8,
- un tarage du capteur, typiquement commandé par le moyen déporté d'affichage et de commande.

L'au moins une carte électronique 15 s'étend parallèlement à l'axe de mesure 10, sous le capteur 8 et sous les moyens de fixation 2.

De manière plus détaillée, l'au moins une carte électronique 15 comprend notamment des sous modules destinés à l'alimentation et à la recharge électrique du dispositif 1, notamment un connecteur 19 (USB) pour la connexion à une source d'alimentation en courant externe au dispositif 1, une batterie, un sous-module de gestion de la batterie, et un sous-module de régulation de l'alimentation de chaque élément du dispositif 1 nécessitant une alimentation.

Une diode 18 indique, lorsqu'elle est allumée (typiquement en vert), un correct niveau de charge de la batterie du dispositif 1.

L'au moins une carte électronique 15 comprend également un microcontrôleur.

Le microcontrôleur comprend avantageusement une mémoire morte reprogrammable EPROM (« Erasable Programmable Read Only Memory »).

Le microcontrôleur est connectable à un ordinateur et au moyen déporté d'affichage par une connexion non filaire (Bluetooth ou Wifi) ainsi que par l'intermédiaire du connecteur 19. Une diode 21 est allumée (typiquement en rouge) lorsque la liaison non filaire est en panne ou présente un problème de connexion avec le moyen déporté d'affichage.

Le module microcontrôleur intègre plusieurs composants et notamment une RAM, une mémoire FLASH et un convertisseur analogique numérique. Ces composants sont configurés de sorte à permettre une calibration des mesures de forces. A cet effet, un programme informatique est chargé dans la mémoire du microcontrôleur de sorte à réaliser la calibration et les mesures du capteur 8.

Un bouton 17 permet d'allumer et d'éteindre le dispositif 1. Une diode 20 est allumée (typiquement en vert) lorsque le dispositif est allumé.

Le dispositif 1 venant d'être décrit permet de multiples mesures de force sur un patient potentiellement très faible, par exemple de force d'extension ou flexion d'un genou ou de l'abduction d'une épaule ou de la flexion d'un coude.

Par exemple, dans le cadre d'une myopathie inflammatoire, la myosite à inclusion, il a été possible de valider **la** qualité du dispositif 1 selon l'invention pour l'extension du genou en comparant les couples de forces estimés à partir de l'appareil isocinétique/isométrique de référence System 3 pro de chez BIODEX et ceux estimés à partir d'un dispositif selon l'invention. Les résultats montrent un agrément absolu excellent entre les deux systèmes de mesure.

Bien sûr, l'invention n'est pas limitée aux exemples qui viennent d'être décrits et de nombreux aménagements peuvent être apportés à ces exemples sans sortir du cadre de l'invention.

Par exemple, dans une variante de ce qui vient d'être décrit, les moyens de fixation comprennent une ventouse ou un aimant ou un collier de serrage ou des moyens de clipsage.

## Revendications

1. Dispositif (1) de mesure d'une force comprenant :
- des moyens de fixation (2) agencés pour fixer le dispositif à un support, les moyens de fixation comprenant des moyens de fixation par serrage comprenant un étau comprenant deux mâchoires (4, 5) agencées pour serrer le support sur lequel est fixé l'étau et/ou une ventouse et/ou un aimant,
- un capteur (8), comprenant un membre de mesure (9) agencé pour se déplacer selon un axe de mesure (10), ledit capteur étant agencé pour mesurer une force exercée sur le membre de mesure en fonction du déplacement de ce membre de mesure, les moyens de fixation étant agencés pour, lors d'un déplacement du membre de mesure le long de l'axe de mesure, exercer sur le support sur lequel ils sont fixés une force moyenne portée par l'axe de mesure
le dispositif comprenant une paroi extérieure (13), le capteur (8) étant situé à l'intérieur de la paroi extérieure de sorte que la paroi extérieure est munie d'un trou de passage (14) faisant face au membre de mesure (9) le long de l'axe de mesure(10), le membre de mesure (9) étant situé sur une face (16) du capteur de sorte que cette face ne soit pas en contact de la paroi extérieure (13).

2. Dispositif selon l'une quelconque des revendications précédentes, **caractérisé en ce que** les moyens de fixation comprennent une ventouse ou un aimant ou un collier de serrage.

3. Dispositif selon l'une quelconque des revendications précédentes, **caractérisé en ce que** le capteur (8) a une précision inférieure ou égale à 0,5N.

4. Dispositif selon l'une quelconque des revendications précédentes, **caractérisé en ce que** le capteur a une plage de mesure d'au moins 0N à 900 N.

5. Dispositif selon l'une quelconque des revendications précédentes, **caractérisé en ce que** le membre de mesure (9) est muni d'un pas de vis agencé pour fixer au membre de mesure un élément extérieur s'étendant le long de l'axe de mesure (10) à travers le trou de passage (14).

6. Dispositif selon l'une quelconque des revendications précédentes, **caractérisé en ce qu'**il comprend au moins une carte électronique (15) agencée pour :
- une mise en forme d'un signal électronique en provenance du capteur en données de mesure de force et/ou
- une alimentation électrique du capteur et/ou d'autres éléments du dispositif, et/ou
- une communication, vers l'extérieur du dispositif, de données de mesure de force en provenance du capteur, et/ou
- un stockage de données de mesure de force en provenance du capteur.

7. Dispositif selon la revendication 6, **caractérisé en ce que** l'au moins une carte électronique (15) s'étend parallèlement à l'axe de mesure (10), sous le capteur (8) et sous les moyens de fixation (2).

8. Ensemble comprenant :
- un dispositif selon la revendication 6 ou 7, dans lequel l'au moins une carte électronique est agencée pour une communication sans fil, vers l'extérieur du dispositif, de données de mesure de force en provenance du capteur, et
- un moyen déporté agencé pour afficher les données de mesure et/ou un traitement des données de mesure, ledit moyen déporté étant relié audit dispositif par une liaison non filaire.

## Patentansprüche

1. Vorrichtung (1) zum Messen einer Kraft, umfassend:
- Befestigungsmittel (2), die zum Befestigen der Vorrichtung an einer Halterung angeordnet sind, wobei die Befestigungsmittel Klemmbefestigungsmittel umfassen, die einen Schraubstock, umfassend zwei Backen (4, 5), die zum Festklemmen der Halterung angeordnet sind, an der der Schraubstock befestigt ist, und/oder einen Saugnapf und/oder einen Magneten umfassen,
- einen Sensor (8), umfassend ein Messorgan (9), das angeordnet ist, um sich entlang einer Messachse (10) zu bewegen, wobei der Sensor angeordnet ist, um eine auf das Messorgan ausgeübte Kraft in Abhängigkeit von der Bewegung dieses Messorgans zu messen, wobei die Befestigungsmittel angeordnet sind, um bei einer Bewegung des Messorgans entlang der Messachse eine von der Messachse übertragene durchschnittliche Kraft auf die Halterung auszuüben, an der sie befestigt sind, wobei die Vorrichtung eine Außenwand (13) umfasst, wobei der Sensor (8) innerhalb der Außenwand positioniert ist, sodass die Außenwand mit einem Durchgangsloch (14) versehen ist, das dem Messorgan (9) entlang der Messachse (10) gegenüberliegt, wobei das Messorgan (9) auf einer Seite (16) des Sensors positioniert ist, sodass diese Seite nicht mit der Außenwand (13) in Kontakt steht.

2. Vorrichtung nach einem der vorstehenden Ansprüche, **dadurch gekennzeichnet, dass** die Befestigungsmittel einen Saugnapf oder einen Magneten oder eine Klemmschelle umfassen.

3. Vorrichtung nach einem der vorstehenden Ansprüche, **dadurch gekennzeichnet, dass** der Sensor (8) eine Genauigkeit von weniger als oder gleich 0,5 N aufweist.

4. Vorrichtung nach einem der vorstehenden Ansprüche, **dadurch gekennzeichnet, dass** der Sensor einen Messbereich von mindestens 0 N bis 900 N aufweist.

5. Vorrichtung nach einem der vorstehenden Ansprüche, **dadurch gekennzeichnet, dass** das Messelement (9) mit einem Gewinde versehen ist, das angeordnet ist, um ein externes Element, das sich entlang der Messachse (10) durch das Durchgangsloch (14) erstreckt, an dem Messelement zu befestigen.

6. Vorrichtung nach einem der vorstehenden Ansprüche, **dadurch gekennzeichnet, dass** sie mindestens eine elektronische Karte (15) umfasst, die angeordnet ist für:
- ein Umwandeln eines elektronischen Signals aus dem Sensor in Kraftmessdaten und/oder
- eine Stromversorgung des Sensors und/oder anderer Elemente der Vorrichtung und/oder
- ein Übermitteln der Kraftmessdaten aus dem Sensor zu dem Äußeren der Vorrichtung und/oder
- ein Speichern der Kraftmessdaten aus dem Sensor.

7. Vorrichtung nach Anspruch 6, **dadurch gekennzeichnet, dass** sich die mindestens eine elektronische Karte (15) parallel zu der Messachse (10) unterhalb des Sensors (8) und unterhalb der Befestigungsmittel (2) erstreckt.

8. Baugruppe, umfassend:
- eine Vorrichtung nach Anspruch 6 oder 7, wobei die mindestens eine elektronische Karte für ein drahtloses Übermitteln von Kraftmessdaten aus dem Sensor zu dem Äußeren der Vorrichtung angeordnet ist, und
- ein entferntes Mittel, das angeordnet ist, um die Messdaten anzuzeigen und/oder für eine Verarbeitung der Messdaten, wobei das entfernte Mittel über eine drahtlose Verbindung mit der Vorrichtung verbunden ist.

## Claims

1. Device (1) for measuring a force comprising:
- fastening means (2) arranged in order to fasten the device to a support, the fastening means comprising fastening means by clamping, comprising a vice comprising two jaws (4, 5) arranged in order to grip the support onto which the vice is attached, and/or a suction cup and/ora magnet
- a sensor (8), comprising a measuring member (9) arranged in order to be displaced along a measurement axis (10), said sensor being arranged in order to measure a force exerted on the measuring member as a function of the displacement of this measuring member,
the fastening means being arranged so that, during a displacement of the measuring member along the measurement axis, they exert on a support on which they are fixed an average force carried along the measurement axis
the device comprising an outer wall (13), the sensor (8) being situated inside the outer wall so that the outer wall is equipped with a through hole (14) facing the measuring member (9) along the measurement axis (10), the measuring member (9) being situated on a face (16) of the sensor so that this face is not in contact with the outer wall (13).

2. Device according to any one of the preceding claims, **characterized in that** the fastening means comprise a suction cup or a magnet or a clamp band.

3. Device according to any one of the preceding claims, **characterized in that** the sensor (8) has an accuracy less than or equal to 0.5 N.

4. Device according to any one of the preceding claims, **characterized in that** the sensor has a measuring range of at least 0 N to 900 N.

5. Device according to any one of the preceding claims, **characterized in that** the measuring member (9) is equipped with a screw thread arranged in order to attach to the measuring member an external element extending along the measurement axis (10) though the through hole (14).

6. Device according to any one of the preceding claims, **characterized in that** it comprises at least one electronic board (15) arranged for:
- shaping an electronic signal originating from the sensor into force measurement data, and/or
- supplying the sensor and/or other elements of the device with electricity, and/or
- communicating, to outside the device, force measurement data originating from the sensor, and/or
- storing force measurement data originating from the sensor.

7. Device according to claim 6, **characterized in that** the at least one electronic board (15) extends parallel to the measurement axis (10), under the sensor (8) and under the fastening means (2).

8. Assembly comprising:
- a device according to claim 6 or 7, in which the at least one electronic board is arranged for wireless communication, to outside the device, of force measurement data originating from the sensor, and
- a remote means arranged in order to display the measurement data and/or to process the measurement data, said remote means being connected to said device by a wireless connection.
